# EUROPEAN PATENT SPECIFICATION

(11) **EP 1 311 672 B1**
(45) Date of publication and mention of the grant of the patent: **01.03.2006**
(21) Application number: 01963639.8
(22) Date of filing: 22.08.2001
(51) Int. Cl.: C12N 15/11, A61P 31/18, A61K 35/12

(54) **tRNA-derived inhibitors of HIV reverse transcriptase**
Inhibitoren der Reversen Transkriptase von HIV aus tRNA
Inhibiteurs de la transcriptase inverse du VIH derivees du tRNA

(30) Priority: 22.08.2000 SE 0003002
(43) Date of publication of application: 21.05.2003
(73) Proprietor: Primert AB, 731 22 Köping (SE)
(72) Inventor: KVIST, Sune, S-731 30 Köping (SE); STRANDBERG, Bror, S-756 52 Uppsala (SE)
(74) Representative: Larsson, Kjell
(86) International application number: PCT/SE2001/001791
(87) International publication number: WO 2002/016608

(56) References cited:
- WO-A1-98/55652
- LEILA SARIH-COTTIN: 'Preferential interaction of human immunodeficiency virus reverse transcriptase with two regions of primer tRNA lys as evidenced by footprinting studies and inhibition with synthetic oligoribonucleotides' J. MOL. BIOL. vol. 226, 1992, pages 1 - 6, XP002905456
- SIMON LITVAK ET AL.: 'Priming of HIV replication by tRNA lys 3: Role of reverse transcriptase' TRENDS IN BIOCHEMICAL SCIENCES vol. 19, no. 3, 1994, pages 114 - 118, XP002905457
- BELINDA B. OUDE ESSINK ET AL.: 'Structural requirements for the binding of tRNA lys 3 to reverse transcriptase of human immunodeficiency virus type 1' THE JOURNAL OF BIOLOGICAL CHEMISTRY vol. 270, no. 40, 1995, pages 23867 - 26874, XP002905458
- RIM EL DIRANI-DIAB ET AL.: 'Phosphorothioate oligonucleotides derived from human immunodeficiency virus type 1 (HIV-1) primer tRNA lys 3 are strong inhibitors of HIV-1 reverse transcriptase and arrest viral replication in infected cells' ANTIMICROBIAL AGENTS OF CHEMOTHERAPY vol. 41, no. 10, 1997, pages 2141 - 2148, XP002905459

### SEQUENCE LISTING

<110> Primert AB
<120> New Sequences
<130> 2018322
<150> SE0003002-3
   <151> 2000-08-22
<160> 7
<170> PatentIn version 3.1
<210> 1
   <211> 76
   <212> RNA
   <213> Rabbit
<220>
   <221> tRNA
   <222> (1)..(76)
   <223>
<220>
   <221> stem_loop
   <222> (10)..(25)
   <223> Dihydrouridine stem-loop
<220>
   <221> stem_loop
   <222> (27) .. (43)
   <223> Anticodon stem-loop
<220>
   <221> stem_loop
   <222> (49) .. (65)
   <223> TpC stem-loop
<220>
   <221> misc_structure
   <222> (1) . (7)
   <223> Basepairing with bases 66-72
<220>
   <221> misc_structure
   <222> (66)..(72)
   <223> Basepairing with bases 1-7
<220>
   <221> source
   <222> (1) .. (76)
   <223> Rabbit liver tRNA Lys3
<220>
   <221> misc_feature
   <222> (10)..(10)
   <223> m2g
<220>
   <221> misc_feature
   <222> (16)..(16)
   <223> d
<220>
   <221> misc_feature
   <222> (20) .. (20)
   <223> d
<220>
   <221> misc_feature
   <222> (27)..(27)
   <223> p
<220>
   <221> misc_feature
   <222> (34).. (34)
   <223> mcm5s2u
<220>
   <221> misc_feature
   <222> (37).. (37)
   <223> ms2t6a
<220>.
   <221> misc_feature
   <222> (39).. (39)
   <223> p
<220>
   <221> misc_feature
   <222> (46).. (46)
   <223> m7g
<220>
   <221> misc_feature
   <222> (47) .. (47)
   <223> d
<220>
   <221> misc_feature
   <222> (48)..(48)
   <223> m5c
<220>
   <221> misc_feature
   <222> (49)..(49)
   <223> m5c
<220>
   <221> misc_feature
   <222> (54)..(54)
   <223> tm
<220>
   <221> misc_feature
   <222> (55)..(55)
   <223> p
<220>
   <221> misc_feature
   <222> (58)..(58)
   <223> m1a
<400> 1
   gcccggauan cucagncggn agagcancag acunuunanc ugaggnnnna gggnncangu 60
   cccuguucgg gcgcca 76
<210> 2
   <211> 35
   <212> RNA
   <213> Artificial Sequence
<220>
   <225> Bases 10-44 in tRNA Lys3. All phosphate ribonucleoside linkages are substituted with phosphorothioate linkages.
<220>
   <221> misc_feature
   <222> (1).. (1)
   <223> m2g
<220>
   <221> misc_feature
   <222> (7) .. (7)
   <223> d
<220>
   <221> misc_feature
   <222> (11)..(11)
   <223> d
<220>
   <221> misc_feature
   <222> (18)..(18)
   <223> p
<220>
   <221> misc_feature
   <222> (25).. (25)
   <223> mcm5s2u
<220>
   <221> misc_feature
   <222> (28)..(28)
   <223> ms2t6a
<220>
   <221> misc_feature
   <222> (30)..(30)
   <223> p
<220>
   <221> stem_loop
   <222> (1)..(16)
   <223>
<220>
   <221> stem_loop
   <222> (18).. (34)
   <223>
<400> 2
   ncucagncgg nagagcanca gacunuunan cugag 35
<320> 3
   <211> 34
   <212> RNA
   <213> Artificial Sequence
<220>
   <223> Bases 10-43 in tRNA Lys3 with standard, "classical" bases instead of modified bases. All phosphate linkages are substituted with phosphorothioate linkages.
<220>
   <221> stem_loop
   <222> (1) .. (16)
   <223>
<220>
   <221> stem_loop
   <222> (18)..(34)
   <223>
<400> 3
<210> 4
   <211> 35
   < 212 > RNA
   <213> Artificial Sequence
<220>
   <223> Bases 10-44 in tRNA Lys3 with standard, "classical" bases instead of modified bases. All phosphate linkages are substituted with phosphorothioate linkages.
<220>
   <221> stem_loop
   <222> (1)..(16)
   <223>
<220>
   <221> stem_loop
   <222> (18)..(34)
   <223>
<220>
   <221> misc_difference
   <222> (1)..(4)
   <223> 2'-O-methyl ribose
<220>
   <221> misc_difference
   <222> (13).. (21)
   <223> 2'-O-methyl ribose
<220>
   <221> misc_feature
   <222> (35)..(35)
   <223> deoxyguanosine
<400> 4
   gcucagucgg uagagcauca gacuuuuaau cugan 35
<210> 5
   <211> 35
   <212> RNA
   <213> Artificial Sequence
<220>
   <223> Bases 10-44 in tRNA Lys3 with a mixture of standard, "classical" bases and modified bases. All phosphate linkages are substituted with phosphorothioate linkages.
<220>
   <221> stem_loop
   <222> (1)..(16)
   <223>
<220>
   <221> stem_loop
   <222> (18) .. (34)
   <223>
<220>
   <221> misc_difference
   <222> (1)..(4)
   <223> 2'-O-methyl ribose
<220>
   <221> misc_difference
   <222> (13)..(17)
   <223> 2'-O-methyl ribose
<220>
   <221> misc_difference
   <222> (19).. (21)
   <223> 2'-O-methyl ribose
<220>
   <221> misc_feature
   <222> (7)..(7)
   <223> d
<220>
   <221> misc_feature
   <222> (11)..(11)
   <223> d
<223>
   <221> misc_feature
   <222> (18).. (18)
   <223> p
<220>
   <221> misc_feature
   <222> (30)..(30)
   <223> p
<220>
   <221> misc_feature
   <222> (35)..(35)
   <223> deoxyguanosine
<400> 5
   gcucagncgg nagagcanca gacuuuuaan cugan 35
<210> 6
   <211> 34
   <212> RNA
   <213> Artificial Sequence
<220>
   <223> Bases 10-43 in tRNA Pro. All phosphate linkages are substituted with phosphorothioate linkages.
<400> 6
   gucuaggggu augauucucg cuuagggugc gaga 34
<210> 7
   <211> 15
   <212> RNA
   <213> Artificial Sequence
<220>
   <223> Bases 28-42 in tRNA Lys3 with standard, "classical" bases instead of modified bases. All phosphate linkages are substituted with phosphorothioate linkages.
<400> 7
   cagacuuuua aucug 15

## Description

### Field of the invention

The present invention relates to new nucleic acid molecules that interact with the reverse transcriptase of a retrovirus. The nucleic acid molecules comprise a sequence composed of two stem-loop structures and a short bridge between the two stems, which molecule for the purposes of the interaction with reverse transcriptase is analogous to the dihydrouridine(D)-stem-loop and anticodon(A)-stem-loop of a mammalian transfer RNA (tRNA). The invention also relates to use of the new nucleic acid molecules as a medicament, as well as use of these molecules for the manufacture of a medicament for the inhibition of the interaction of HIV-1 and HIV-2 reverse transcriptase with tRNA^{Lys3}. The invention further relates to a method of inhibiting the interaction of reverse transcriptase of HIV-1 and HIV-2 with tRNA^{Lys3}.

### Background of the invention

Retroviruses are believed to cause approximately 1.6-2 percent of all human malignancies worldwide (Blattner, W.A., *Proc. Assoc. Am*. *Physicians* (1999) **111**(6), 563-72). Retroviruses utilise a complex mechanism to reverse transcribe their RNA genome into double-stranded DNA, for subsequent insertion into the genomic DNA of the host cell. In the first step, the single-stranded RNA genome is copied to a single-stranded DNA by means of a virally encoded enzyme called reverse transcriptase (RT). This polymerase has the ability to interact with both the RNA strand of the viral genome (RNAᵥ) and the tRNA molecule, forming a ternary complex which initiates reverse transcription of the RNAᵥ. The two RNA molecules (RNAᵥ and tRNA) interact with different entities of the RT molecule. The polymerisation is initiated from the part of the tRNA that is hybridised to the primer binding site (PBS) of RNAᵥ. The tRNA used is acquired from the invaded host. The first 18 nucleotides of the 3' end of the tRNA are complementary to the PBS of the RNAᵥ. The 3' end of the tRNA is the starting point for the polymerisation, which proceeds to the 5' end of the RNAᵥ by incorporation of nucleotides. A DNA strand complementary to part of the RNAᵥ strand is the primary result.

In addition to exogenous retroviruses known to infect humans, there have been characterised endogenous retroviruses. These retroviruses, incorporated into the genome and propagated to subsequent generations, are believed to constitute approximately 0.5% of the human genome (Bieda, K., Hoffmann, A., Boller, K., *J. Gen. Virol.* (2001), **82** (3) , 591-6) Other retroviruses have been studied, for which it is not known whether they are endogenous or not. One such example is a proviral structure in some human breast cancers, a 9.9 kb provirus with 95% homology to the mouse mammary tumour virus, MMTV (Liu, B., Wang, Y., Melana, S.M., Pelisson, I., Najfeld, V., Holland, J.F., Pogo, B.G., *Cancer Research* (2001), **61**, 1754-1759).

One of the most studied retroviruses is the human immunodeficiency virus (HIV). The reason is the efforts to try to find drugs to cure the serious illness AIDS, which is caused by the retroviruses HIV-1 and HIV-2 which infect and kill a certain type of T lymphocytes (CD4⁺), dendritic cells and macrophages. Based on the knowledge of the RNA/DNA transcription mechanism of the HIV virus, several so called nucleotide analogues of the type AZT are used as inhibition drugs today. Chemically, these nucleotides lack the 3' hydroxy group which is needed for the incorporation of the following nucleotide during DNA synthesis. When such a nucleotide is built into the sequence, the polymerisation will be terminated and no viral DNA is formed. The infection stops. HIV reverse transcriptase lacks the type of proof-reading which is built into many other polymerases. This means that the mutation frequency during polymerisation is extremely high, resulting in new mutated RT molecules that avoid incorporation of the analogues. Resistance to the analogue, and thus to the drug, is obtained. With this type of therapy, it is then necessary to use several different nucleotide analogues at the same time, to minimise the risk of resistance. The current therapy is effective in restoring CD4⁺ T lymphocytes that die during active HIV infection. 90-99% of the lymphocytes can be restored during a treatment period of 3-12 months. It is, however, impossible to stop the treatment, since HIV also replicates in cells such as macrophages and dendritic cells, which grow, and thus replicate the virus, very slowly. These cells will then serve as a type of reservoir for the virus, and the patient will quickly return to a state of high virus content in the blood, followed by depletion of the restored CD4⁺ T lymphocyte pool, when the treatment is stopped. Thus, there is a great need for a treatment that is effective also for cells characterised by slow replication of the virus.

Several mutants of RT, resistant to nucleoside analogue inhibitors as well as to non-nucleoside analogue inhibitors, are known to arise and impede the treatment of AIDS (Antivirals Against AIDS, eds Unger, R.E., Kreuter, J., Rabsamen-Waigmann, H., (2000) Marcel Dekker, Basel, p 80). There is consequently a need for inhibitors which are effective against mutations that occur during currently available therapy.

One such approach to circumventing resistance would be to block the specific interactions between the tRNA and RT molecules. A reverse transcriptase, which is inhibited by a blocking agent able to interact with those amino acid residues of RT that otherwise specifically bind the primer tRNA molecule, will, through mutations of those residues, become resistant to that blocking agent only while simultaneously losing its capacity to bind tRNA, and thus will no longer be able to function. In other words, a mutation through which the RT molecule escapes the inhibition of the blocking agent will also rob the RT molecule of its ability to use tRNA as primer for DNA synthesis .

The polymerase RT, and its complex with RNAᵥ and tRNA, has been the subject of much investigation. Especially the RT of HIV-1 and HIV-2, and its stable complex with its cognate tRNA, which is tRNA^{Lys3}, has been intensively studied. The region of tRNA^{Lys3} involved in the interaction between HIV-1 RT and tRNA^{Lys3} has been studied by digestion of the primer with pancreatic ribonuclease in the presence and absence of HIV-1 RT. It has been shown that the enzyme HIV-1 RT protects a tetranucleotide of the anticodon loop and a trinucleotide of the dihydrouridine loop of tRNA^{Lys3} (Sarih-Cottin, L., Bordier, B., Musier-Forsyth, K., Andreola, M-L., Barr, P.J., Litvak, S., *J. Mol. Biol.* (1992) **226**, 1-6). In the same reference, different synthetic oligoribonucleotides, corresponding to the anticodon stem and loop or the dihydrouridine stem and loop of tRNA^{Lys3}, were produced separately, and it was shown that these nucleotides inhibit HIV-1 RT, showing interaction between these tRNA regions and HIV-1 RT.

In another work, the inhibition of HIV-RT by DNA fragments in phosphorothioate form with sequences corresponding to tRNA^{Lys3} acceptor stem or anticodon stem and loop was investigated (El Dirani-Diab, R., Sarih-Cottin, L., Delord, B., Dumon, B., Moreau, S., Toulme, J-J., Fleury, H., Litvak, *S., Antimicrobial Agents* and *Chemotherapy,* (1997), 41, No.10, 2141-2148). Both ordinary DNA nucleotides and oligonucleotides in phosphorothioate form were produced. In both cases the nucleotide sequences each corresponded to a different secondary structure element of tRNA^{Lys3}. According to the reference, no inhibition was observed when ordinary DNA nucleotides were used, even at a concentration as high as 40 µM. With the DNA nucleotides in phosphorothioate form, inhibition of the HIV-1 RT was observed.

A therapy based on these findings is so far not available. It is accordingly an object of the present invention to provide a nucleic acid molecule with a high affinity for interacting with reverse transcriptase of a retrovirus. Another object is to provide a nucleic acid molecule with a sufficiently high capacity for blocking the access of primer molecules to reverse transcriptase of a retrovirus. A molecule which interacts with HIV-1 and HIV-2 reverse transcriptase is especially an object of the invention.

Another object of the invention is a nucleic acid molecule, which interacts with reverse transcriptase of a retrovirus, for use as a medicament.

A further object of the invention is use of a nucleic acid molecule, which interacts with reverse transcriptase of a retrovirus, for the manufacture of a medicament for the inhibition of the interaction of HIV-1 and HIV-2 reverse transcriptase with tRNA^{Lys3}.

### Summary of the invention

The objects of the invention are obtained by the nucleic acid molecule as claimed in the claims. According to the invention there is provided a nucleic acid molecule, which interacts with reverse transcriptase of a retrovirus, comprising a nucleotide sequence composed of two stem-loop structures and a short bridge between the two stems, which molecule for the purposes of the interaction with reverse transcriptase is analogous to the dihydrouridine (D) -stem-loop and' anticodon (A)-stem-loop sequences of a mammalian transfer RNA (tRNA). Said sequence presents at least parts of the following tRNA structural elements: D-stem strand 1, D-loop, D-stem strand 2, a bridge of 1 - 3 bases, A-stem strand 1, A-loop, A-stem strand 2. In the backbone of said nucleic acid molecule, some or all of the normal phosphodiester linkages can be substituted with phosphorothioate linkages. A preferred nucleic acid molecule of the invention has, in analogy to a mammalian tRNA, said structural elements in order from the 5' end: D-stem strand 1, D-loop, D-stem strand 2, a bridge of 1 - 3 bases, A-stem strand 1, A-loop, A-stem strand 2. Preferably, the nucleic acid molecule has a non-paired base as the last base.

According to another aspect of the invention, the nucleic acid molecule identified above is used as a medicament.

A further aspect of the invention is use of the identified nucleic acid molecule for the manufacture of a medicament for the inhibition of the interaction of HIV-1 and HIV-2 reverse transcriptase with tRNA^{Lys3}.

A method of inhibition of the interaction of reverse transcriptase of HIV-1 and HIV-2 with tRNA^{Lys3} is also presented. The method comprises administering to a human in need of such treatment an amount of a nucleic acid molecule as identified above that is efficient for the inhibition.

All retroviruses known to replicate share the mechanism of being able to utilise host cell tRNA to prime DNA synthesis on their RNA genome. All mammalian tRNAs share the same basic fold, and the common two-dimensional representation of the Watson-Crick base pairing pattern is known as a "cloverleaf" structure, containing between 73 and 93 ribonucleotides. The genetic sequences are highly conserved between species, and the sequences of tRNA^{Lys3} genes are identical between creatures as diverse as rabbits and humans. The cloverleaf structure/base pairing pattern is highly conserved within mammalian tRNAs. Consequently, the procedure (the mechanism) of obstructing those surfaces of a reverse transcriptase that interact with the tRNA anticodon and dihydrouridine stem-loops, by bringing into interaction with those surfaces a nucleic acid molecule, which has a sequence and/or structure which is analogous to the D-stem-loop and A-stem-loop of a mammalian tRNA, which has the correct separation of loops; and contains the conserved bases of natural tRNA loops, will be generally applicable.

Compared to the state of the art, the present molecule has the advantages of being a molecule long enough to have two main binding points to the reverse transcriptase instead of one for the known shorter fragments.

It is well known that nucleic acid molecules, especially RNA molecules, can be very unstable. According to the present invention, it was also surprisingly found that it is possible to synthesise a long, stable RNA molecule containing both the D-stem-loop and A-stem-loop by substituting some or all of the phosphate groups with phosphorothioate groups in the ribonucleotide linkages.

### Figure legends

Figure 1: Cloverleaf structure of natural tRNA^{Lys3}.
Figure 2: RP-HPLC fractionation profile of the crude synthetic 35-mer phosphorothioate ribonucleotide (SEQ ID NO:4) corresponding to nucleotides 10-44 in Figure 1.
Figure 3: Analysis by RP-HPLC of pooled fractions of SEQ ID NO:4.
Figure 4: Test of binding of SEQ ID NO:3 to Mut1, using gel filtration on a SMART™ system (Amersham Pharmacia Biotech); a: Mut1 only, b: mixture of Mut1 and SEQ ID NO:3.
Figure 5: Binding of Mut1 to SEQ ID NO:3, shown on polyacrylamide gel electrophoresis (PAGE with PhastGel system from Amersham Pharmacia Biotech).
Figure 6: Binding of Mut1 to SEQ ID NO:4, using gel filtration on a SMART™ system. a: Mut1 only, b: mixture of Mut1 and SEQ ID NO:4, c: corresponding measurements for a series of ratios of SEQ ID NO:4 : Mut1 ranging from 0 to 6.5.
Figure 7: Binding of Mut1 to SEQ ID NO:4, shown on polyacrylamide gel electrophoresis (PAGE with PhastGel system from Amersham Pharmacia Biotech).
Figure 8: IC₅₀ values for SEQ ID NO:5 were determined using varying concentrations of either enzyme (A), primer (odT) (B) or dNTP substrate (BrdUTP) (C). The RT reaction time used was 3 hours.

### Detailed description of the invention

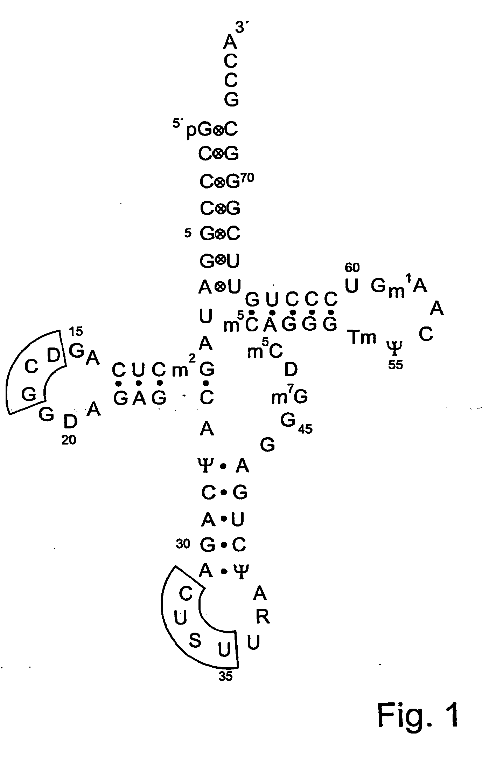

The nucleic acid molecule according to the present invention preferably comprises 34-35 nucleotides, although shorter or longer variants are possible. The nomenclature for the nucleic acid molecule as identified above is analogous to conventional nomenclature for natural tRNA (see Figure 1). The naming starts from the 5' end with the first strand of the stem leading to the dihydrouridine loop. This is D-stem strand 1. Then comes the dihydrouridine loop followed by D-stem strand 2. The D-stem strand 2 is connected by a bridge of 1-3 bases to the first strand of the stem leading to the anticodon loop. This latter strand is named A-stem strand 1. Then follow the anticodon loop and A-stem strand 2. At the end of the sequence there can be a non-paired base. In a preferred embodiment this base is a DNA base.

The present invention anticipates DNA as well as RNA molecules, and also chimeric oligonucleotides where some bases are DNA and others are RNA. The backbone of the oligonucleotides of the invention may well consist of standard phosphodiester linkages in their entirety, but in preferred embodiments, some or all of these have been replaced by phosphorothioate linkages (see below). The rationale when designing an oligonucleotide according to the present invention takes into consideration factors like the molecule's stability and resistance to degradation by enzymes, as well as the binding geometry of the molecule to its reverse transcriptase target. These considerations will lead to oligonucleotides with different compositions, both in terms of DNA/RNA content and in terms of the proportion of phosphodiester/phosphorothioate linkages. The creation of proper chimeric RNA/DNA molecules with modified backbones according to the invention is well within the grasp of the skilled man, once the principles of the present invention have been elucidated.

Note that, for the purposes of the invention, only those structural elements or parts thereof necessary for interaction of the nucleic acid molecule of the invention with the viral reverse transcriptase need be included, as long as said nucleic acid molecule retains sufficient stability. Therefore, the invention is also assumed to encompass variants, in which the number of nucleotides in the two stem-loop elements or in the linking bridge is not exactly that of the natural tRNA. As long as the nucleic acid molecule presents functional equivalents to the two stem-loop structures described above, it falls within the scope of the invention. Variants that are contemplated as embodiments of the invention also, for example, comprise cyclic permutations of the nucleic acid molecules described herein, in which the order of the different stem-loop sequences is altered but the secondary structure remains intact. Furthermore, the invention is also assumed to encompass variants where an extension to A-stem strand 2 is complementary to one or more bases in the bridge described above.

The molecules of the invention are useful for inhibiting the interaction of reverse transcriptase of a retrovirus. In the present context, "retroviruses" are intended to encompass both exogenous and endogenous retroviruses.

"Interaction" of the nucleic acid molecule of the invention with reverse transcriptase is meant to include stable binding of the molecules to each other, as well as any interactions leading to the cleavage or disintegration of any of the two species as a result of the interaction.

A characterising feature of tRNA is that it contains unusual bases, e.g. derivatives of adenine (A), uracil (U), cytosine (C) and guanine (G). According to a preferred embodiment of the present invention, these naturally occurring derivatives are used to maximise the likeness of the nucleic acid molecules of the invention to the tRNA elements of interest. However, the synthesis of molecules comprising such unusual derivatives is more complicated and costly than the synthesis of molecules consisting entirely of the "classical" bases A, C, G, U/T. Thus, it may be preferable in certain cases to use such classical bases for reasons of economy or ease of synthesis.

To stabilise further the nucleic acid molecule according to the invention, in the case that this molecule is an RNA molecule, the ribose in one or more of the nucleotides in D-stem strand 1, D-stem strand 2 and A-stem strand 1 is preferably replaced by 2'-O-methyl ribose. Furthermore, it is preferred to replace the ribose in the bridge connecting D-stem strand 2 with A-stem strand 1 with 2'-O-methyl ribose. In an especially preferred embodiment of this RNA molecule, the ribose in all of the nucleotides in D-stem strand 1, D-stem strand 2 and A-stem strand 1 is replaced by 2'-O-methyl ribose, as well as in the bridge. The addition of a methyl group in the positions mentioned makes the molecule less sensitive to hydrolysis. Interestingly and surprisingly, the reinforcement through introduction of 2'-O-methyl ribose at these particular locations yields a molecule which is resistant to degradation in a particularly beneficial fashion.

The present invention is especially applicable to tRNA^{Lys3}, which is the tRNA used for priming of DNA polymerisation by HIV-1 and HIV-2 reverse transcriptase. The whole sequence of natural tRNA^{Lys3} contains 76 bases and is set out in SEQ ID NO:1 in the sequence listing. According to an embodiment of the present invention, an RNA molecule of 35 bases corresponding to bases no. 10 - 44 in tRNA^{Lys3} is obtained, but with all phosphodiester linkages substituted with phosphorothioate linkages. The sequence of this RNA molecule is set out in SEQ ID NO:2.

In a preferred embodiment of the invention, an RNA molecule corresponding to bases no. 10 - 43 in tRNA^{Lys3} is produced in which, beside the phosphorothioate linkages, the naturally occurring nucleotide derivatives have been substituted with the corresponding unmodified A, G, C and U nucleotides. The sequence of this molecule is set out in SEQ ID NO:3. In another preferred embodiment, set out in SEQ ID NO:4, the molecule as set out in SEQ ID NO:3 is modified in bases 1-4 and 13-21 in such a way that 2'-O-methyl ribose is substituted for normal ribose. The numbering of the sequence in SEQ ID NO:4 starts from base no.1 in SEQ ID NO:3. The methylated bases are the bases in D-stem 1 and 2, the bridge bases and the bases in A-stem 1. In the sequence as set out in SEQ ID NO:4, a further unpaired base has been added which is a deoxyribose base, so that this nucleotide is a DNA nucleotide. The two molecules with sequences SEQ ID NO:3 and SEQ ID NO:4 are used in binding experiments under experimental section A below.

A particularly preferred molecule according to the invention is a molecule which is similar in sequence to SEQ ID NO:4, but which incorporates some naturally occurring nucleotide derivatives to make it more analogous to tRNA^{Lys3}. Furthermore, in this molecule the 2'-O-methyl ribose of position 18 is changed back to a normal ribose. The sequence of this molecule is set out in SEQ ID NO:5, and is used in comparative experiments under experimental section B below.

According to the invention an RNA molecule as set out in SEQ ID NO:6, which interacts with HTLV-1 and HTLV-2 reverse transcriptase is also provided. This sequence corresponds to bases 10-43 in natural tRNA^{Pro}, but with phosphorothioate linkages.

The invention will be further illustrated by the following non-limiting examples.

### A. Studies of complex formation between nucleic acid molecules of the invention and an RNase H inactive mutant of HIV-1 RT

### Experimental procedure

### Example A1

First, a synthetic molecule that should interact with purified RT from HIV-1, and form a complex stable enough to be visualised by biochemical methods, is produced.

**Chemistry**. Phosphoramidite chemistry is used to synthesise an RNA 34-mer similar, but not identical, in sequence to the sequence in the tRNA^{Lys3}, which interacts with the RT. Transfer RNA molecules contain a number of modified nucleotides, which are derivatives of the common, unmodified nucleotides. Comparing the tRNA^{Lys3} sequence with the synthetic 34-mer, the modified nucleotides are replaced as follows: position 1 (N2-methyl-guanosine in tRNA^{Lys3} , guanosine in the 34-mer) ; positions 7 and 11 (dihydrouridine in tRNA^{Lys3} , uridine in the 34-mer); positions 18 and 30 (pseudouridine in tRNA^{Lys3}, uridine in the 34-mer) ; position 25 [5-(methoxycarbonylmethyl)-2-thio-uridine in tRNA^{Lys3}, uridine in the 34-mer]; and finally position 28 [C2-methylthio-N6-(threoninecarbonyl)-adenosine in tRNA^{Lys3}, adenosine in the 34-mer].

**Synthesis**. Synthesis is performed on an Applied Biosystems Division (ABD) DNA/RNA Synthesizer Model 394 at the synthesis scale of 1.0 *µ*mol. Final yield of crude oligonucleotide is 232 nmol. All monomer nucleotides are ribonucleotides with the 2'-hydroxyl group protected by Fpmp (Reese, C.B., Thomson, E.A., *J. Chem*. *Soc. Perkin Trans. 1* (1988), 2281). The exocyclic amines on the nucleosides riboadenosine and ribocytidine are protected by benzoyl (bz) groups, whereas the amino group on the riboguanosine is protected by isobutyryl (isobu). The ribouridine nucleoside does not carry any amino group that it is necessary to protect. All monomer nucleotides are protected at their 5'-hydroxyl group by a dimethoxytrityl (DMT) group, and the phosphorous atom is protected by a cyanoethyl group. The sequence synthesised is set out in SEQ ID NO:3.

The common phosphodiester bonds between nucleotides are all replaced by phosphorothioate bonds, thus replacing one of the oxygen atoms on the phosphorous atom by a sulphur atom. This modification of the oligonucleotide backbone makes it resistant to most ribonucleases, thereby facilitating laboratory work with the oligonucleotide.

Synthesis is performed in the 3' to 5' direction with the extreme 3' riboadenosine nucleotide covalently linked to the solid support Controlled Pore Glass (CPG; pore size 1000 Å). The following additional reagents are used for the individual synthesis steps:
(i) Removal of the 5' DMT protecting group is achieved by treatment with 3% dichloroacetic acid (DCA) dissolved in anhydrous (< 50 ppm water) dichloromethane (DCM). This solution (DCA/DCM) is pulsed into the column containing the CPG for 5-8 x 12 s, with 5 s waiting steps between the deliveries. The reaction is interrupted by extensive rinsing with anhydrous (< 10 ppm water) acetonitrile.
(ii) Elongation of the oligonucleotide (coupling reaction) is performed by simultaneous addition of the correct phosphoramidite nucleotide (concentration 100 mM in anhydrous acetonitrile) and the activator 5-ethyl-thio-1*H*-tetrazole (concentration 500 mM in anhydrous acetonitrile). The molar excess of amidite over CPG-bound nucleotide is 20-fold. The activator/amidite solution is delivered in two portions with a waiting step of 400 s in between, followed by another waiting step of 200 s. The reaction is terminated by an argon gas rinse, followed by extensive rinsing with anhydrous acetonitrile.
(iii) Oxidation of the phosphorous atom is done by a 90 s reaction with a 50 mM solution of Beaucage reagent in anhydrous acetonitrile. Again, the reaction is terminated by an argon gas rinse, followed by extensive rinsing with anhydrous acetonitrile.
(iv) Termination of oligonucleotide chains which do not couple the latest base added is obtained by reaction with a mixture of acetic anhydride/pyridine/tetrahydrofuran and N-methylimidazole/tetrahydrofuran (capping reaction). The reaction is terminated after 12 s by an argon gas rinse, followed by extensive rinsing with anhydrous acetonitrile.

The reaction steps described above constitute the "synthesis cycle", and are repeated until the oligonucleotide's complete sequence is obtained.

**Cleavage and deprotection** is performed by transfer of the CPG with its bound oligonucleotide to a glass vessel with a teflon-lined screw cap. Ammonia solution (35%) is added (1.0 ml), and is allowed to cleave the oligonucleotide from the CPG for 60-90 min at room temperature (20-25°C). The vial is then transferred to an oven at 55-60°C, and is incubated for another 12-18 h in order to remove the base protecting groups (bz and isobu).

The solution is chilled to +4°C, and low molecular weight molecules are removed by gel filtration through Sephadex G-25 (DNA grade) equilibrated in MilliQ 18.2 MΩ water containing 0.1% diethylpyrocarbonate (DEPC water). The oligonucleotide is eluted into a 4.5 ml polypropylene tube, and is lyophilised for 12-18 h.

The dry material is resuspended in 1.0 ml of a low pH buffer (water/pyridine/formic acid; pH 2-3) to remove the 2'-Fpmp protecting group. The ribooligonucleotide does not dissolve in this solution, but forms a precipitate until deprotected. Thus, removal of the 2'-Fpmp group makes the ribooligonucleotide soluble. The mixture is vortexed at high frequency and allowed to react until the precipitate is completely dissolved (24-36 h). The solution is neutralised by addition of 200 *µ*l of 0.5 M sodium carbonate, and low molecular weight molecules are immediately removed by gel filtration through Sephadex G-25 (DNA grade) equilibrated in DEPC water. The oligonucleotide is eluted in 1.8 ml into an amber glass vial which has been rinsed and heated to 55-60°C in DEPC water. An aliquot is removed and diluted for A₂₆₀ measurement and determination of the final amount of the ribooligonucleotide.

The remaining ribooligonucleotide is lyophilised to dryness during 16-24 h.

After reconstitution in a water-based buffer, the ribooligonucleotide is used for binding experiments to RT of HIV-1 virus.

### Example A2:

In this example a 35-mer chimeric RNA/2'-O-methyl-RNA/DNA molecule with sequence homology to a part of the human tRNA^{Lys3} molecule is synthesised.

To exclude or minimise the interference of non-full-length molecules present in the crude synthesis mixture, the target 35-mer molecule is purified by High Performance Liquid Chromatography (HPLC; see below).

**Chemistry**. Essentially the same chemistry as described in example A1 is used to synthesise the 35-mer (see Example A1; Chemistry). However, the following important changes are introduced to improve the quality of the synthesis:
(i) Synthesis is performed on the same instrument as previously, but at the synthesis scale of 15.0 *µ*mol.
(ii) The activator used is 4,5-dicyanoimidazole at a concentration of 0.8 M in anhydrous acetonitrile.
(iii) The molar excess of phosphoramidite nucleotides over CPG-bound nucleotide is 10-fold only.
(iv) The deoxyguanosine nucleotide is added at the 3'-end.
(v) The following 2'-hydroxyl RNA nucleotides are replaced by their 2'-O-methyl counterparts: 1 through 4, and 13 through 21. The sequence is set out in SEQ ID NO:4.

As previously, all internucleotide bonds are phosphorothioate linkages.

**The synthesis** cycle is changed accordingly to the increase in synthesis scale from 1.0 to 15.0 *µ*mol. The details are as follows:
(i) Removal of the 5' DMT protecting group is done by treatment with 3% dichloroacetic acid (DCA) dissolved in anhydrous (< 50 ppm water) dichloromethane (DCM). This solution (DCA/DCM) is pulsed into the column containing the CPG for 4 x 30 s, and 3 x 20 s. There are no waiting steps between the deliveries. The reaction is interrupted by extensive rinsing with anhydrous (< 10 ppm water) acetonitrile.
(ii) Elongation of the oligonucleotide (coupling reaction) is performed by the simultaneous addition of the correct phosphoramidite nucleotide (concentration 100 mM in anhydrous acetonitrile) and the activator 4,5-dicyanoimidazole (concentration 800 mM in anhydrous acetonitrile). The molar excess of the amidite over CPG-bound nucleotide is 10-fold. The activator/amidite solution is delivered in five 7-s portions with a waiting step of 10 s in between, followed by a waiting step of 200 s after the last delivery. The reaction is terminated by an argon gas rinse, followed by extensive rinsing with anhydrous acetonitrile.
(iii) Oxidation of the phosphorous atom is done by a 4x 20 s delivery of the 50 mM solution of Beaucage reagent in anhydrous acetonitrile. Again, the reaction is terminated by an argon gas rinse, followed by extensive rinsing with anhydrous acetonitrile.
(iv) Termination of oligonucleotide chains that do not couple the latest base added is obtained by reaction with a mixture of acetic anhydride/pyridine/tetrahydrofuran and N-methylimidazole/tetrahydrofuran (capping reaction). The reaction is performed by 2x 30 s delivery with a waiting step of 20 s in between, and is terminated by an argon gas rinse, followed by extensive rinsing with anhydrous acetonitrile.

**Cleavage and deprotection** is performed by transfer of the CPG with its bound oligonucleotide to a 50 ml glass bottle with a teflon-lined screw cap. Ammonia solution (35%) is added (15.0 ml), and is allowed to cleave the oligonucleotide from the CPG for 60-90 min at room temperature (20-25°C). The vial is then transferred to an oven at 55-60°C, and is incubated for 14-18 h in order to remove the base protecting groups (bz and isobu).

The solution is chilled to +4°C, and low molecular weight molecules are removed by gel filtration through Sephadex G-25 (DNA grade) equilibrated in MilliQ 18.2 MΩ water containing 0.1% diethylpyrocarbonate (DEPC water).

Elution of the oligonucleotide is done by taking fractions, each of 4.0 ml, and by analysing 10 *µ*l from each by A₂₆₀ determination and anion exchange HPLC.

Fractions containing substantial amounts of target oligonucleotide are pooled (giving a total of 32.0 ml), and divided into two equal portions in 50 ml Falcon tubes. The material is lyophilised for 36-48 h.

One of the two tubes containing dried ribooligonucleotide is resuspended in 4.0 ml of a low pH buffer (water/pyridine/formic acid; pH 2-3), to remove the 2'-Fpmp protecting group. The mixture is vortexed at a high frequency and allowed to react for 48 h. The solution is neutralised by addition of 1.0 ml of 0.5 M sodium carbonate, and low molecular weight molecules are immediately removed by gel filtration through Sephadex G-25 (DNA grade) equilibrated in DEPC water. The oligonucleotide is eluted in 6.0 ml into an amber glass vial, which has been rinsed and heated to 55-60°C in DEPC water. An aliquot is removed and diluted. for A₂₆₀ measurement, and the final amount is determined to constitute 918 nmol. This is lyophilised to dryness during 24 h.

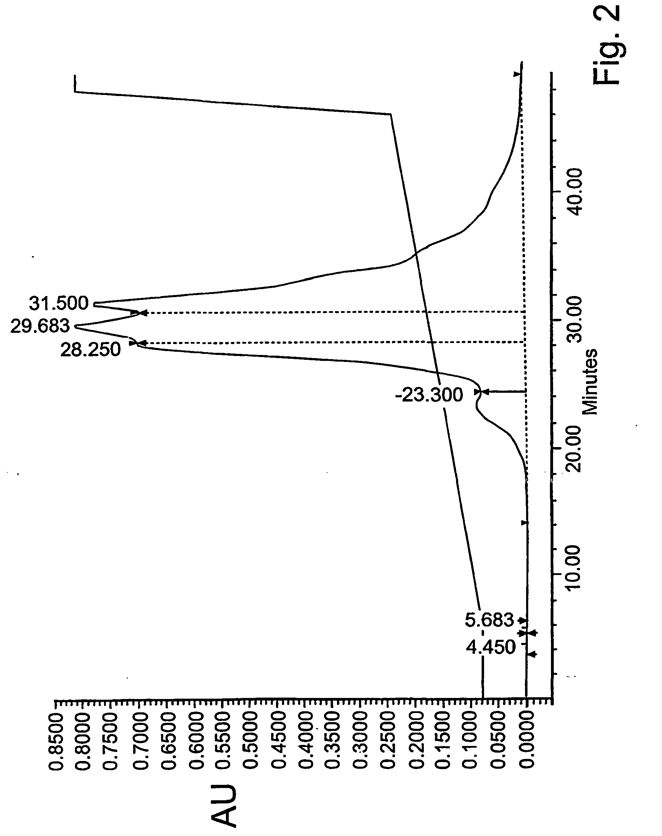

**Purification** is performed by Reverse Phase HPLC (RP-HPLC) on a Waters Delta 4000 system. The ribooligonucleotide material is dissolved in 50 mM triethylamine acetate (TEAA), pH 8.0, in DEPC-treated MilliQ 18.2 MΩ water, and is fractionated on an Amberchrom column (CG 300s; 25 x 250 mm). The fractionation profile is shown in Figure 2. Collection of fractions is started at time 19.45 and stopped at 38.45 min after injection. Elution is performed by buffers A (0.1 M TEAA, pH 7.0) and B (0.1 M TEAA, pH 7.0, 80% acetonitrile).

Approximately 60 fractions (5.0 ml per fraction) are collected, the fractions 20-51 covering the main peak.

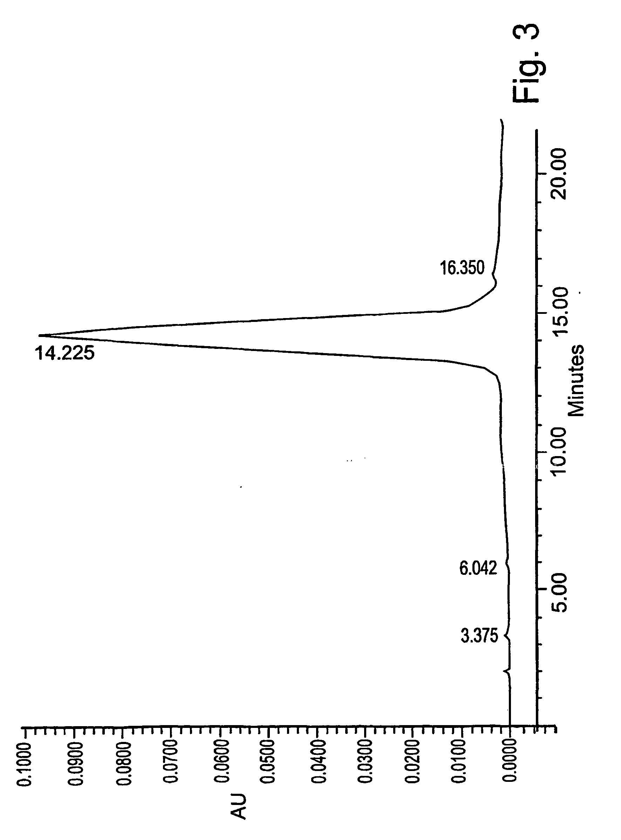

**Analysis** is done by RP-HPLC (Column Waters µBondapak C₁₈, 3.9 x 150 mm). Aliquots from fractions containing the target molecule are pooled in different combinations, and these pools are analysed in the same way as the individual fractions. Fractions 24-33 are found to give the highest purity, and these fractions are pooled. Analysis of this pool gave an almost symmetrical peak with a purity of 99.7%, as shown in Figure 3. As the phosphorothioate internucleotide bonds may have the sulphur atom positioned to the phosphorous atom in two alternative ways (see below) it is unlikely that a peak would appear in a chromatogram as completely symmetrical. 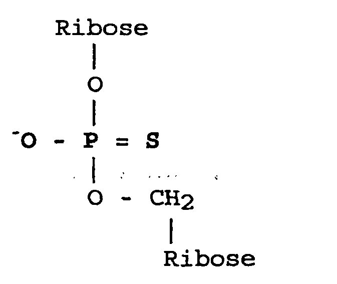 or, alternatively:

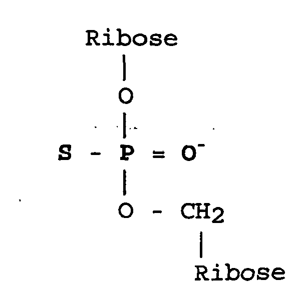

The amount obtained of the ribooligonucleotide is divided into five equal portions, each containing 500 µg in amber 10 ml vials, and lyophilised. The dried product is sealed under argon.

The following data are calculated for this oligonucleotide:

| | |
|---|---|
| Molecular weight: | 11918.38 *µ*g per µmol |
| Absorbance coefficients: | 30.66 *µ*g per A₂₆₀-unit and ml |
| | 388.73 A₂₆₀-units per *µ*mol |
| Final yield after purification: | 265 nmol, corresponding to 3158.37 *µ*g |
| Purity by RP-HPLC: | 99.7% |

A retain containing 658.37 *µ*g is kept frozen at -20°C.

### Example A3:

In this example, a series of 35-mer or 34-mer chimeric RNA/2'-O-methyl-RNA/DNA molecules according to the invention are presented. Some of these comprise modified nucleotides corresponding to the authentic nucleotides present in the sequence of natural human tRNA^{Lys3} molecule.

**Chemistry**. The tRNA^{Lys3} molecule contains a large number of unusual ribonucleotides normally not found in mRNA or rRNA. These modified nucleotides are derivatives of the common nucleotides. In the cases below where the molecule according to the invention comprises such modified nucleotides, the modified ribonucleotides can be synthesised in the form of phosporamidites in order to be useful for the same type of chemistry as that described in Example A2. In total, 8 different molecules according to the invention are presented, each one with a unique structure:
- A 35-mer where D-stem strand 1, dihydrouridine loop, D-stem strand 2, A-stem strand 1, anticodon loop and A-stem strand 2 are RNA with a phosphorothioate backbone. The bridging sequence between D-stem strand 2 and A-stem strand 1 is DNA with a phosphorothioate backbone.
- A 35-mer where D-stem strand 1, dihydrouridine loop, anticodon loop and A-stem strand 2 are RNA with a phosphorothioate backbone. D-stem strand 2, the bridging sequence between D-stem strand 2 and A-stem strand 1, and A-stem strand 1 are DNA with a phosphorothioate backbone.
- A 34-mer where D-stem strand 1, D-stem strand 2, the bridging sequence between and A-stem strand 1, A-stem strand 1 and A-stem strand 2 are DNA with a phosphorothioate backbone. Dihydrouridine loop and anticodon loop are RNA with a phosphorothioate backbone.
- A 35-mer where D-stem strand 1, dihydrouridine loop, D-stem strand 2, the bridging sequence between D-stem strand 2 and A-stem strand 1, and A-stem strand 1 are DNA with a phosphorothioate backbone. Anticodon loop and A-stem strand 2 are RNA with a phosphorothioate backbone.
- A 35-mer where D-stem strand 1, D-stem strand 2, A-stem strand 1 and A-stem strand 2 are DNA. The bridging sequence between D-stem strand 2 and A-stem strand 1, the dihydrouridine loop and the anticodon loop are phosphorothioate DNA.
- A 35-mer where the entire molecule is DNA with a phosphorothioate backbone. Positions 7 and 11 are dihydrouridine and positions 18 and 30 are pseudouridine.
- A molecule in which D-stem strand 1, dihydrouridine loop, D-stem strand 2, A-stem strand 1, anticodon loop and A-stem strand 2 are RNA with a phosphorothioate backbone. The bridging sequence between D-stem strand 2 and A-stem strand 1 is 2'-O-methyl modified phosphorothioate RNA.
- A molecule in which D-stem strand 1, dihydrouridine loop, anticodon loop and A-stem strand 2 are RNA with a phosphorothioate backbone. D-stem strand 2, A-stem strand 1 and the bridging sequence between D-stem strand 2 and A-stem strand 1 are 2'-O-methyl modified phosphorothioate RNA. Positions 7 and 11 are dihydrouridine and positions 18 and 30 are pseudouridine. Position 25 is 5-(methoxycarbonylmethyl)-2-thio-uridine and position 28 is C2-methylthio-N6-(threoninecarbonyl)-adenosine.

**Synthesis** of the modified ribonucleotides can be performed on an Applied Biosystems Division (ABD) DNA/RNA Synthesizer Model 394 under the same conditions as described in Example A2. A synthesis scale of 15.0 µmol will be adequate.

**Purification** can be performed by Reverse Phase-HPLC (RP-HPLC) on a Waters Delta 4000 system as described in Example A2. Purification and analysis methods, essentially the same as described in Example A2, will be appropriate.

### Binding testing of the synthesised nucleic acid molecules

In the following experiment, the terms "34-mer" and "35-mer" refer to SEQ ID NO:3 (produced in example A1 above) and SEQ ID NO:4 (produced in example A2 above), respectively. In order to show that the nucleic acid molecules synthesised above interact with reverse transcriptase of HIV-1, two different experiments are performed. Natural RT molecules have an RNase H activity which normally cleaves RNA molecules. To avoid this problem, an RNase H inactive mutant of HIV-1 RT is used in the experiments. This mutant is produced according to the method given in Schatz, O., Cromme, F.V., Gruninger-Leitch, F. and Le Grice, S.F.J., *Febs Letter.* Vol.**257**, No.2 p.311-314. This RT-mutant is further on referred to as Mut1. The following experiments are carried out:
1. Test of binding by means of separation of the complex Mut1 - 34-mer (from Example A1) from excess 34-mer using gel filtration on a SMART system.
2. Gel mobility shifts in polyacrylamide gel electrophoresis (PAGE) experiments.

### Test of the 34-mer of Example A1:

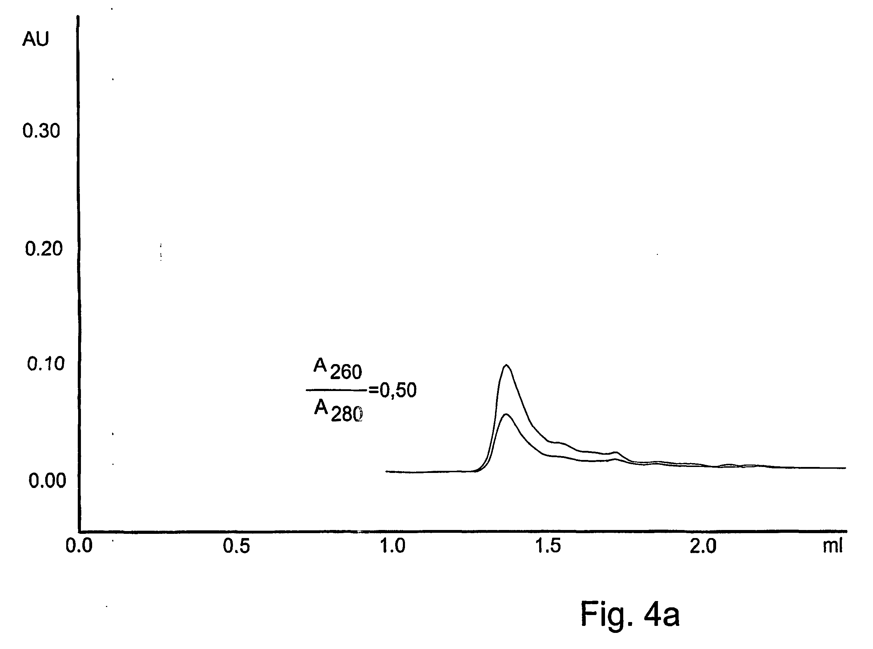

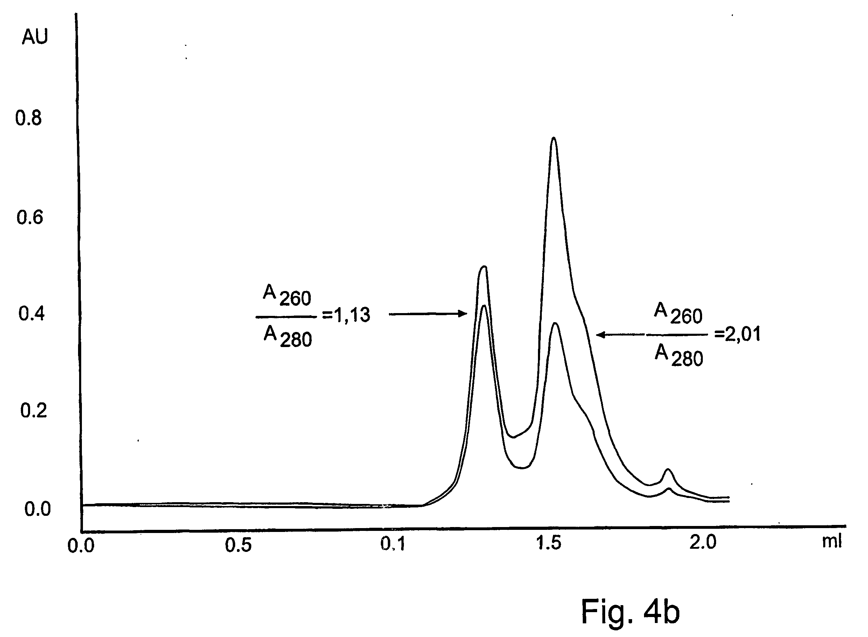

1. Mutl is incubated with excess 34-mer for about 90 min at 4°C, buffer Tris-HCl, pH=8.0, containing 5 mM MgCl₂. The mixture is then applied to a gel filtration column of a SMART™-type system, which allows simultaneous registration at two different wavelengths. The result is shown in Figures 4a and 4b. Figure 4a shows the absorption for Mut1 only: The absorption at 260 nm (the lower curve) is lower than the absorption at 280 nm (the upper curve) : A₂₆₀/A₂₈₀ = 0.50, which is an expected value for a protein.
   In Figure 4b, the absorption for the mixture of Mutl and 34-mer is shown:
   Starting amounts: Mut1 = 210 pmol; 34-mer = 630 pmol. The left peak is the complex Mut1 - 34-mer. The absorption at 260 nm (the upper curve) is now higher than the absorption at 280 nm (the lower curve) : A₂₆₀/A₂₈₀ = 1.13. The right peak is the excess 34-mer, which has the characteristic absorption ratio for nucleic acids: A₂₆₀/A₂₈₀ = 2.01. By integration of the peak for excess 34-mer (see Figure 4b) and calibration against the peak when running 34-mer only, it is possible to determine the amount of 34-mer present in the complex. The result was 34-mer : Mut1 = 0.9 : 1.00 in the complex. With starting amounts of Mut1 = 210 pmol and 34-mer = 1350 pmol (excess 6.5 : 1), the complex contained 34-mer : Mut1 in a ratio of about 1.00 : 1.00.
2. Binding of Mut1 to 34-mer has been shown with native polyacrylamide gel electrophoresis.

a) Native 20% PAGE gel is run at 4-5°C. Samples are incubated at 4°C in 50 mM hepes buffer and 5 mM magnesium chloride and varying salt concentrations as indicated below.

| | |
|---|---|
| Lane 1: | 34-mer |
| Lane 2: | 34-mer in 1.5 M ammonium sulphate and 0.3 M potassium chloride |
| Lane 3: | Mut1 in 1.5 M ammonium sulphate and 0.3 M potassium chloride |
| Lane 4: | Mut1 with 34-mer in 1.5 M ammonium sulphate and 0.3 M potassium chloride |
| Lane 5: | Mut1 with 34-mer in 1.6 M ammonium sulphate |
| Lane 6: | Mut1 with 34-mer in 1.5 M ammonium sulphate and 0.3 M potassium chloride |
| Lane 7: | Mut1 with 34-mer in 1.6 M ammonium sulphate |
| Lane 8: | Mut1 with 34-mer |

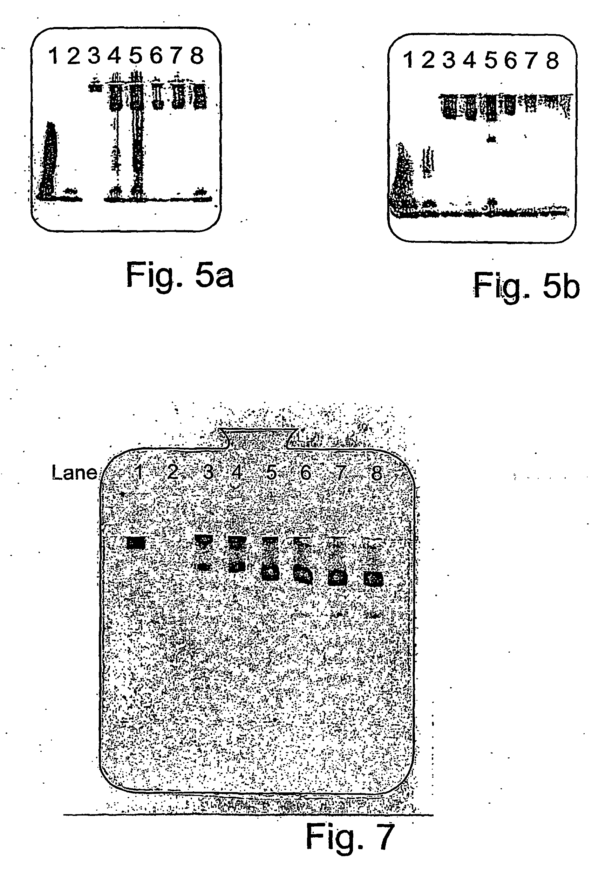

Samples are incubated for 90 min at 4°C prior to a further 10 min incubation at 4°C with ammonium sulphate and/or KCl Samples applied to lanes 4 and 5 have twice the amount of 34-mer used for the other samples.
Mut1 in complex with 34-mer migrates faster than Mut1 alone as shown in Figure 5a (lane 4 with 34-mer and lane 3 without).
b) Native 20% PAGE gel run at 4-5°C. Samples are incubated at 4°C in 50 mM hepes buffer and 5 mM magnesium chloride, with varying concentrations of ammonium sulphate.

| | |
|---|---|
| Lane 1: | 34-mer |
| Lane 2: | 34-mer in 1.6 M ammonium sulphate |
| Lane 3: | Mut1 with 34-mer and 1.6 M ammonium sulphate |
| Lane 4: | Mut1 with 34-mer in 0.8 M ammonium sulphate |
| Lane 5: | Mut1 with 34-mer |
| Lane 6: | Mut1 with 15-mer |
| Lane 7: | Mut1 with 15-mer in 0.8 M ammonium sulphate |
| Lane 8: | Mut1 with 15-mer in 1.6 M ammonium sulphate |

Degradation products accompanying the 34-mer are shown to have a similar gel mobility as a phosphorothioate 15-mer (SEQ ID NO:7) with the base sequence of tRNA^{Lys3} anticodon stem and loop (Figure 5b). At high salt concentrations the 15-mer (anticodon stem and loop) dissociates from Mut1, while the 34-mer (anticodon stem and loop + dihydrouridine stem and loop) remains in complex with Mut1.

### Test of the 35-mer of Example A2:

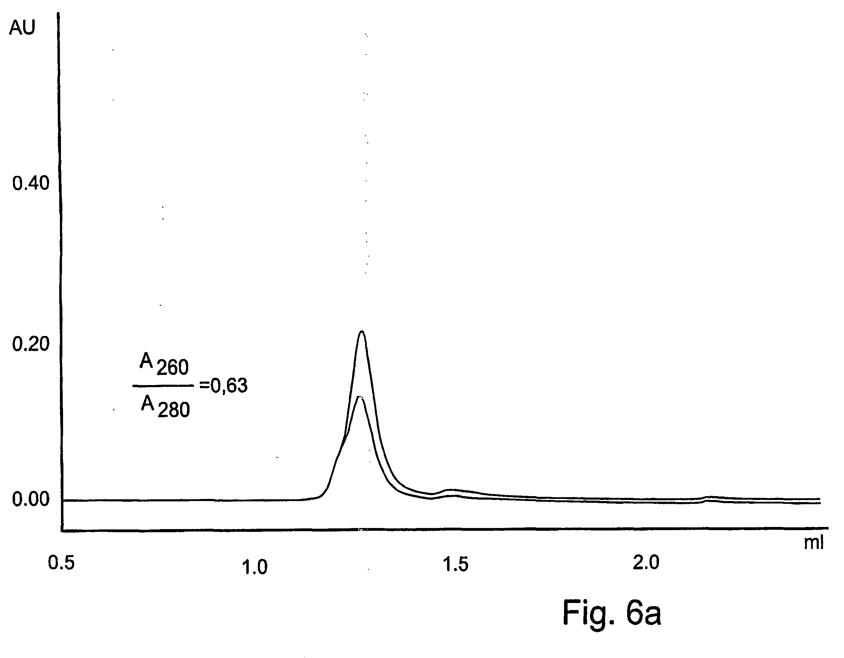

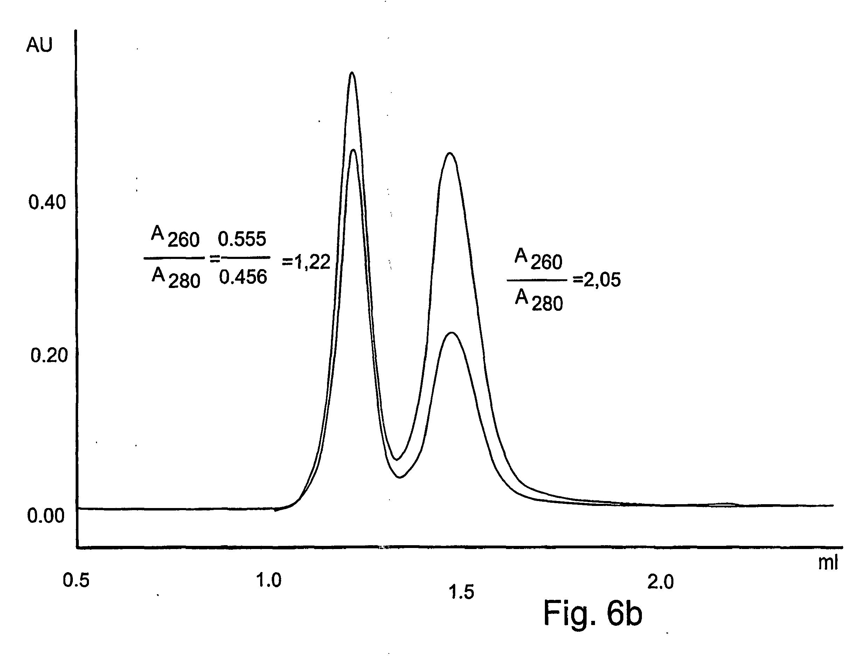

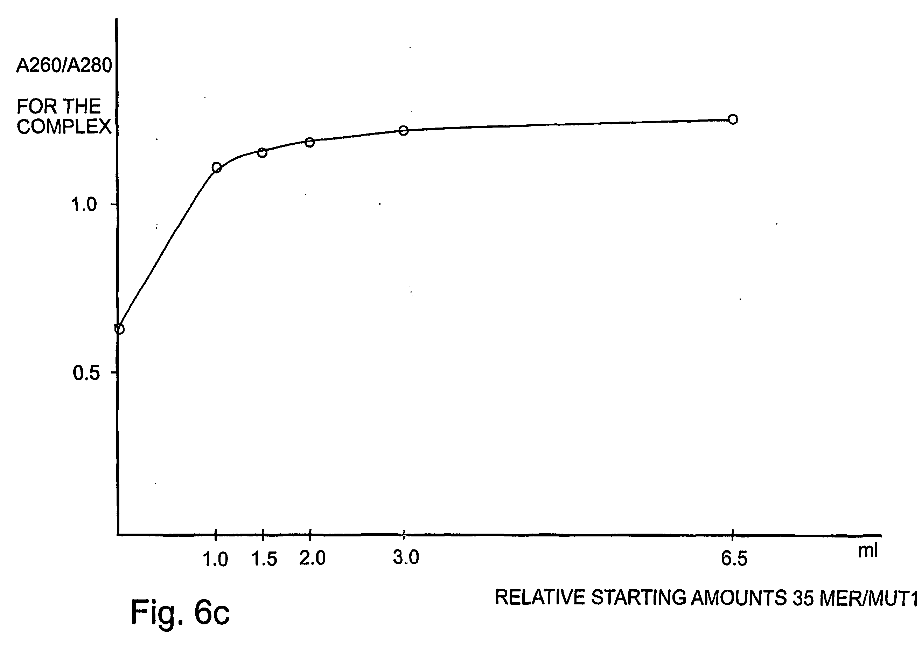

1. Mut1 is incubated with excess 35-mer for about 2 h at 4°C, buffer Tris-HCl, pH=8.0, containing 5mM MgCl₂. The mixture is then applied to a gel filtration column of a SMART™-type system, which allows simultaneous registration at two different wavelengths. The result is shown in Figures 6a, 6b and 6c. Figure 6a shows the absorption for Mut1 only.
The absorption at 260 nm (lower curve) is lower than the absorption at 280 nm (upper curve) : A₂₆₀/A₂₈₀ = 0.63, which is an expected value for a protein.
In Figure 6b, the absorption for the mixture of Mutl and 35-mer is shown: Starting amounts: Mut1 = 210 pmol; 35-mer = 630 pmol. Ratio of 35-mer : Mut1 = 3 : 1
The left peak is the complex Mut1 - 35-mer. The absorption at 260 nm (upper curve) is now higher than the absorption at 280 nm: A₂₆₀/A₂₈₀ = 1.22. The right peak is the excess 35-mer, which has the characteristic absorption ratio for nucleic acids: A₂₆₀/A₂₈₀ = 2.05.
Corresponding measurements have been performed for a series of ratios 35-mer : Mut1 ranging from 0 to 6.5 (see Figure 6c). From Figure 6c it is evident that A₂₆₀/A₂₈₀ as a function of starting amounts 35-mer : Mut1 reaches a plateau, i.e. a situation of saturation, at starting amounts of 35-mer : Mutl of about 3 : 1.
By integration of the peak for excess 35-mer (see Figure 6b) and calibration against the peak when running 35-mer only, it is possible to determine the amount of 35-mer present in the complex. The result is 35-mer : Mut1 = 1.05 : 1.00 in the complex, when the relative starting amounts 35-mer : Mut1 are 3 : 1. This indicates a small amount of a secondary site for 35-mer together with the almost saturated primary site, and this is also observed from the gel mobility shifts (see below).
2. Binding of Mut1 to 35-mer has been shown with native polyacrylamide gel electrophoresis (PAGE).
Native 20% PAGE gel is run at 4-5°C. Samples are incubated at 4°C in 50 mM hepes buffer, 15 mM manganese sulphate, 1 mM EDTA and 0.25 mM EGTA.
Samples are incubated 10 min at room temperature prior to a 60 min incubation at 4°C. One µl of sample solution is applied per lane. Electrophoresis is conducted with up to 400 volts while cooling to approximately 4°C. After 0.209 kWh the gel is silver stained.

| | |
|---|---|
| Lane 1: | Mut1, 1.5 pmol/µl |
| Lane 2: | 35-mer, 15.0 pmol/µl |
| Lane 3: | Mut1, 1.5 pmol/µl, with 35-mer, 1.26 pmol/µl, molar ratio 1 : 0.84 |
| Lane 4: | Mut1, 1.5 pmol/µl, with 35-mer, 2.52 pmol/µl, molar ratio 1 : 1.68 |
| Lane 5: | Mut1, 1.5 pmol/µl, with 35-mer, 3.36 pmol/µl, molar ratio 1 : 2.24 |
| Lane 6: | Mut1, 1.5 pmol/µl, with 35-mer, 5.04 pmol/µl, molar ratio 1 : 3.36 |
| Lane 7: | Mut1, 1.5 pmol/*µ*l, with 35-mer, 6.72 pmol/*µ*l, molar ratio 1 : 4.48 |
| Lane 8: | Mut1, 1.5 pmol/µl, with 35-mer, 8.40 pmol/*µ*l, molar ratio 1 : 5.60 |

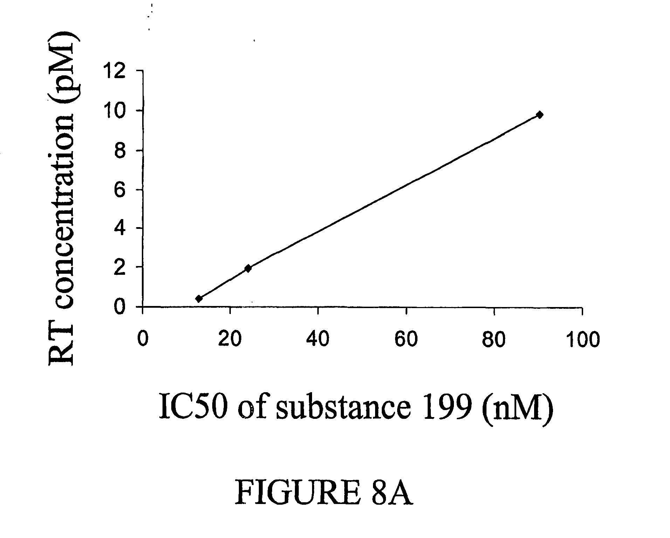

Mut1 in complex with 35-mer migrates faster than Mut1 alone, as shown in Figure 7. At high concentrations of starting amounts of 35-mer (35mer : Mut1 >= 2.25 : 1), a minor fraction of the protein migrates even faster, indicative of a secondary site for 35-mer interaction of low affinity. This is expected for reverse transcriptase which in addition to tRNA anticodon recognition also must interact with RNAᵥ and DNA, as well as recognise and digest DNA/RNA hybrids and cleave off tRNA primers subsequent to their extension.

### B. Inhibition by nucleic acid molecules of the invention of binding of primer to reverse transcriptase

In this series of experiments, the inhibitory action of a nucleic acid molecule according to the invention was demonstrated. Furthermore, comparisons with the inhibitory action of a nucleic acid molecule related to the previously known tRNA analogue fragments and that of a known anti-HIV drug were performed. The nucleic acid molecule of the invention was a 35-mer RNA molecule having the sequence set out in SEQ ID NO:5, which for the purposes of identification in the examples below has been designated "compound 199". A 15-mer RNA molecule, having the sequence set out in SEQ ID NO:7, was designated "compound 198". Compounds 198 and 199 were synthesised in accordance to the methods set forth above in section A. The known anti-HIV drug used is Nevirapine, a non-nucleoside inhibitor of reverse transcriptase which is a member of the dipyridodiazepinone chemical class of compounds.

The inhibitor substances were tested against a panel of recombinant HIV-1 RT:s, of which one served as a reference and the others displayed mutations conferring different types of resistance to known inhibitors (see example B1 below).

### Methods

### Protocol for determination of RNA dependent DNA polymerisation

The flourimetric RT assay (Cavidi® Lenti RT activity kit), available from Cavidi Tech, Uppsala, Sweden, was used for the determination of RT activity. In short, poly(rA) covalently bound to the wells of a 96 well microtiter plate serves as template for the incorporation of 5-bromodeoxyuridine 5'-triphosphate (BrdUTP) during the reverse transcription step at 33°C. The amount of bromodeoxyuridine monophosphate (BrdUMP) incorporated into DNA is detected with an alkaline phosphatase (Ap) conjugated, anti-BrdU monoclonal antibody. An Ap substrate, 4-methylumbelliferyl phosphate, is finally used for fluorimetric product detection.

### Protocol for DNA polymerase assay useful for detection of second strand DNA synthesis by retrovirus RT

The flourimetric DNA-polymerase assay used is available from Cavidi Tech AB, Uppsala, Sweden. It is based on a short DNA primer analogous to a part of the tRNA^{Lys3} sequence. The template is a single stranded deoxynucleotide template with part of the sequence complementary to the primer. The enzyme reaction is dependent on all four deoxynucleotide bases. The thymidine triphosphate is, however, replaced by BrdUTP. The amount of BrdUMP incorporated into DNA during the polymerase reaction is detected with an alkaline phosphatase (Ap) conjugated anti-BrdU monoclonal antibody. An Ap substrate, 4-methylumbelliferyl phosphate, is finally used for fluorimetric product detection.

### Protocols for determination of inhibitor susceptibility of the RT activity

(i) Protocol for determination of inhibition of RNA dependent DNA polymerisation: The inhibition studies were performed in a modified Lenti RT assay. The inhibitors were serially diluted in five steps, whereupon enzyme dilution was added and the enzyme/inhibitor mixtures were incubated for 30 minutes atw33°C. The enzyme reaction was initiated by addition of 100 µl aliquots of the enzyme/inhibitor mixture to each well of a microtiter plate containing 100 µl RT reaction mixture per well. The polymerase reaction was allowed to proceed for 3 hours at 33°C, whereupon the reaction was terminated by a wash of the plate. The IC₅₀ value was defined as the concentration of inhibitor giving 50% inhibition of the polymerase activity studied. The amount of RT used was standardised to an activity corresponding to 90-130 pg (7-10 x 10⁻¹⁶ mol) reference HIV-1 RT per well
**(ii) Protocol for determination of inhibition of second strand synthesis on a variable DNA template**: The inhibition studies were performed in a modified DNA polymerase assay. The inhibitors were serially diluted in five steps, enzyme dilution was added and the enzyme/inhibitor mixtures were incubated for 30 minutes at 33°C. The enzyme reaction was initiated by addition ofw 75 *µ*l aliquots of the enzyme/inhibitor mixture to each well of a microtiter plate containing 75 *µ*l DNA polymerase reaction mixture per well. The polymerase reaction was allowed to proceed for 3 hours at 33°C whereupon the reaction was terminated by a wash of the plate. The IC₅₀ value was defined as the concentration of inhibitor giving 50% inhibition of the polymerase activity studied. The amount of RT used was standardised to an activity corresponding to 40-60 pg (3.3-5.0 x 10⁻¹⁶ mol) reference HIV-1 RT per well.
**(iii) Protocol for RT binding inhibition (BIC) assay:** The study was performed in a modified DNA polymerase assay. The inhibitors were serially diluted in five steps, enzyme dilution was added and the enzyme/inhibitor mixtures were incubated for 30 minutes at 33°C. Aliquots of the enzyme/inhibitor mixture were transferred to each well of a microtiter plate containing either immobilised primer, or primer/template. The enzyme was allowed to bind to the immobilised polymer for 90 min at 33°C. The amount of RT used was standardised to an activity corresponding to 40-60 pg (3.3-5.0 x 10⁻¹⁶ mol) reference HIV-1 RT per well. The plates were then washed to remove unbound enzyme whereupon the amount of bound enzyme was determined by addition of the appropriate reaction solution for polymerisation (i e a reaction solution devoid of primer for studies of primer binding and a reaction solution devoid of both primer and template for studies of binding to primed template). The polymerase reaction was allowed to proceed for 3 hours at 33°C, whereupon the reaction was terminated by a wash of the plate. The BIC₅₀ value was defined as the concentration of inhibitor giving 50% inhibition of the binding of the polymerase activity studied.

### Examples

### Example B1. Substance 199 is an inhibitor of HIV-1 RT reverse transcription

The capacity of the indicated substances to inhibit the enzymes in a panel of recombinant HIV 1 subtype B RTs was studied according to "Protocol for determination of inhibition of RNA dependent DNA polymerisation" (protocol (i) above). The final nucleotide substrate (BrdUTP) concentration was 0.35 µM, and the primer (odT₂₂) amount was 0.03 ng per well. As shown in table 1, substance 199 exhibited the lowest IC₅₀ values of the three inhibitors investigated. The inhibition profiles were similar for all RTs studied (IC₅₀ 6.0-12 nM). Therefore, substance 199 inhibited RTs with amino acid substitutions, known to be associated with resistance to nucleoside (T215Y) and non-nucleoside (L100I, K103N, L101/K103N) RT inhibitors, equally efficient as it inhibited the wild type RTs (reference RT and E478Q) . The IC₅₀ values found for substance 198 were more variable and 50-100 times higher (340-1100 nM) than the IC₅₀ values for substance 199 (see table 1).

**TABLE 1. Effect of inhibitors on reverse transcription performed by recombinant HIV-1 RT:s with defined mutations**

| | IC₅₀ of indicated inhibitor in RT assay (nM) | | | |
|---|---|---|---|---|
| RT type | 198^{a} | 199^{b} | Nevirapine | 199^{b,c} |
| ref RT^{d} | 470 | 6.6 | 3400 | ND^{e} |
| E478Q | 320 | 6.0 | 23000 | 11 |
| L100I | 450 | 10 | 55000 | 7.4 |
| K103N/E478Q | 1100 | 10 | >1000000 | 9.8 |
| L100I/K103N | 900 | 12 | >1000000 | ND^{e} |
| T215Y | 340 | 11 | 21000 | 9.3 |

| | | | | |
|---|---|---|---|---|
| ^{a}SEQ ID NO:7 | | | | |
| ^{b}SEQ ID NO:5 | | | | |
| ^{c}The RT and the inhibitor were added directly to the assay mixture without preincubation (see protocol (i)) | | | | |
| ^{d} HIV type B (clone BH 10) | | | | |
| ^{e} ND = not done | | | | |

The effect of substance 199 on the RT panel was also tested according to an alternative procedure in which the inhibitor was added directly to the RT reaction mixture. The results found were not significantly different from those obtained with the standard procedure. This proves the efficiency of substance 199 in inhibiting first strand synthesis without any pre-incubation period.

### Example B2. Substance 199 is an inhibitor of HIV RT second strand DNA synthesis

The capacity of the indicated substances to inhibit each enzyme in a panel of recombinant HIV 1 subtype B RTs was studied according to "Protocol for determination of inhibition of second strand synthesis on variable DNA template" (protocol (ii) above). The results, which are summarised in table 2, were rather similar to those found for inhibition of reverse transcription (table 1). The four enzymes investigated all showed strikingly different sensitivity to inhibition with the well known non-nucleoside RT inhibitor Nevirapine, which was included as a control in the experiment. Substance 199 exhibited the lowest IC₅₀ values of the three inhibitors investigated. The inhibition profiles for this inhibitor were virtually identical for all RTs studied (IC₅₀ 36-51 nM) . The IC₅₀ values found for substance 198 were more variable and at least one order of magnitude higher (550-1000 nM).

**TABLE 2. Effect of inhibitors on second strand DNA synthesis performed by recombinant HIV-1 RT:s with defined mutations**

| | IC₅₀ of indicated inhibitor in DNAp assay (nM) | | |
|---|---|---|---|
| RT type | 198^{a} | 199^{b} | Nevirapine |
| ref RT^{c} | 550 | 40 | 360 |
| E478Q | 1000 | 34 | 2500 |
| L100I | 220 | 36 | 10000 |
| K103N/E478Q | 1000 | 51 | >1000000 |

| | | | |
|---|---|---|---|
| ^{a} SEQ ID NO: 7 | | | |
| ^{b} SEQ ID NO:5 | | | |
| ^{c} HIV type B (clone BH 10) | | | |

### Example B3. Substance 199 inhibi ts the binding of HIV RT to primer /template

The capacity of the indicated substances to inhibit the binding to primer or to primed DNA template of a panel of recombinant HIV 1 subtype B RTs was studied according to "Protocol for RT binding inhibition (BIC) assay" (protocol (iii) above). The results are depicted in table 3. Substance 199 had the capacity to prevent RT binding to either primer alone or to primed template, whereas substance 198 had significantly less capacity to prevent RT binding. Furthermore, substance 199 was active at much lower concentrations than substance 198. The BIC₅₀ values for substance 199 varied from 2 nM for binding of reference RT to primer to 16 nM for binding of RT L100I to primed template. The corresponding figures for substance 198 was 100 and >1000 nM. Note that all BIC₅₀ values for substance 199 are significantly lower than the corresponding IC₅₀ values for inhibition of second strand DNA synthesis (table 2). The well known non nucleoside RT inhibitor Nevirapine was at all conditions studied unable to prevent RT binding.

**TABLE 3. Effect of inhibitors on the binding of recombinant HIV-1 RT:s with defined mutations to DNA primers or primed DNA templates**

| | Inhibition of binding to primer (BIC₅₀ nM) | | | Inhibition of binding to template/primer (BIC₅₀ nM) | | |
|---|---|---|---|---|---|---|
| RT type | 198^{a} | 199^{b} | Nevirapine | 198^{a} | 199^{b} | Nevirapine |
| ref RT^{c} | 100 | 2 | >1000000 | 900 | 7 | >1000000 |
| E478Q | >1000 | 7 | >1000000 | >1000 | 14 | >1000000 |
| L100I | >1000 | 7 | >1000000 | >1000 | 16 | >1000000 |
| K103N/E478Q | 400 | ND^{d} | >1000000 | 1000 | 16 | >1000000 |

| | | | | | | |
|---|---|---|---|---|---|---|
| ^{a} SEQ ID NO:7 | | | | | | |
| ^{b} SEQ ID NO:5 | | | | | | |
| ^{c} HIV type B (clone BH 10) | | | | | | |
| ^{d} ND = not done | | | | | | |

### Example B4. The inhibitory effect of substance 199 is not dependent on the concentration of dNTP substrate

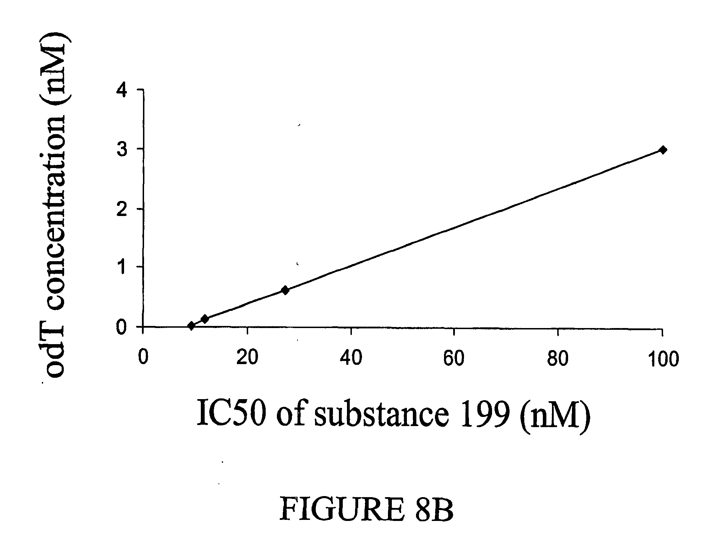

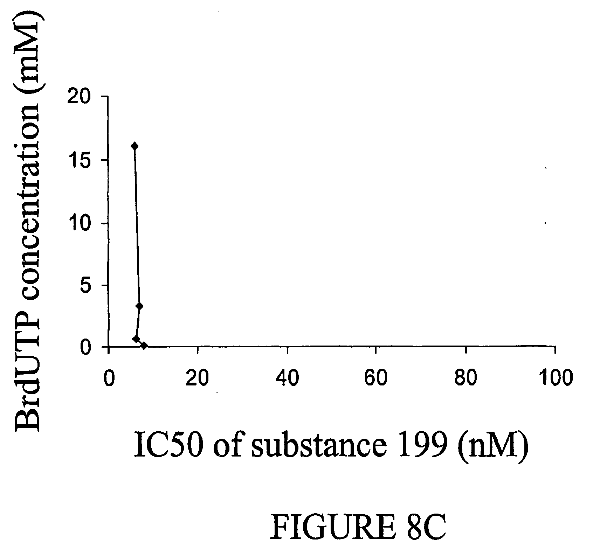

The IC₅₀ values for substance 199 were determined according to "Protocol for determination of inhibition of RNA dependent DNA polymerisation" (protocol (i) above), using varying concentrations of either enzyme, primer (odT) or dNTP substrate (BrdUTP). The RT reaction time used was 3 hours and the results are depicted in Figure 8 (A-C) .

8A) RT E478Q at the indicated concentrations was incubated for 30 minutes at 33°C with serial dilution sets of substance 199. The enzyme reaction was started by addition of 100 *µ*l of the substance/enzyme mixture to microtiter plates with a RT reaction mixture containing 16 *µ*M BrdUTP and 3nM odT.

8B) RT E478Q (concentration 2.5 pM) was incubated for 30 minutes at 33°C with a serial dilution set of substance 199. The enzyme reaction was started by addition of 100 µl samples of the substance/enzyme mixture to microtiter plates with dilution sets of RT reaction mixture containing the indicated concentration of odT primer and 0.35 *µ*m dNTP substrate (BrdUTP).

8C) RT E478Q (concentration 2.5 pM) was incubated for 30 minutes at 33°C with a serial dilution set of substance 199. The enzyme reaction was started by addition of 100 *µ*1 samples of the substance/enzyme mixture to microtiter plates with dilution sets of RT reaction mixture containing the indicated concentration of the dNTP substrate (BrdUTP) and 24 pM odT.

From Figure 8, it can be concluded that the inhibitory effect of substance 199 is influenced by the concentration of enzyme and primer (Figure 8A and 8B) but not by the concentration of dNTP substrate (Figure 8C).

### C. Conclusions

The synthetic nucleic acid molecules produced to exemplify the present invention have several advantages compared to those fragments corresponding to tRNA^{Lys3} that have been described in the prior art. The present molecules are fragments that are considerably longer and have the correct binding geometry. The molecules are 34-35-mers; containing both of the important binding areas, the anticodon loop and stem as well as the dihydrouridine loop and stem. They are phosphorothioate molecules with good stability against hydrolysis by nucleases. Furthermore, nucleic acids in phosphorothioate form bind considerably better to proteins than do the corresponding normal nucleic acids. Those RNA molecules according to the invention that have 2'-O-methyl ribose in 12 or 13 nucleotides are especially stable against hydrolysis. This product shows an almost symmetrical peak when applied to a gel filtration column.

In the experiments carried out under section B above, the molecules according to the invention have IC₅₀ and BIC₅₀ values that are about 50-100 times lower than corresponding values for a 15-meric RNA anticodon stem-loop structure containing phosphorothioate. This 15-mer (corresponding to SEQ ID NO:7) is analogous to previously described anticodon stem-loop structures containing DNA. However, the 15-mer used for this comparison is an RNA phosphorothioate and is therefore more like tRNA than the DNA structures of the prior art.

As mentioned above, the presented mechanism for blocking of the specific parts of the interaction of tRNA with RT has a great potential to be used also against other retroviruses. Another important group of retroviruses is human T-cell leukaemia viruses, HTLV-1 and HTLV-2, which cause leukaemia and neurological disturbances in human beings. A drug that will effectively block the binding of the natural primer tRNA^{Pro} to HTLV-1 and HTLV-2 would be of great medical importance.

## Claims

1. A synthetic nucleic acid molecule, which interacts with reverse transcriptase of a retrovirus, the nucleotide sequence of said molecule being composed of two stem-loop structures and a short bridge between the two stems, which molecule for the purposes of the interaction with reverse transcriptase is analogous to the dihydrouridine(D)-stem-loop and anticodon(A)-stem-loop of a mammalian transfer RNA (tRNA), comprising the following structural elements: D-stem strand 1, D-loop, D-stem strand 2, a bridge of 1 - 3 bases, A-stem strand 1, A-loop, A-stem strand 2; and, optionally, in which molecule some or all of the normal phosphodiester nucleoside linkages have been substituted with phosphorothioate linkages.

2. A nucleic acid molecule according to claim 1, wherein the sequence, in analogy to a mammalian tRNA, has the following structure from the 5'end: D-stem strand 1, D-loop, D-stem strand 2, a bridge of 1 - 3 bases, A-stem strand 1, A-loop, A-stem strand 2.

3. A nucleic acid molecule according to claim 2, wherein the sequence further contains a non-paired base linked to the end of A-stem strand 2.

4. A nucleic acid molecule according to any one of the preceding claims, which is an RNA molecule.

5. An RNA molecule according to claim 4, wherein the ribose is replaced by 2'-O-methyl ribose in at least one of the nucleotides in D-stem strand 1, D-stem strand 2 and A-stem strand 1.

6. An RNA molecule according to claim 5, wherein all of the ribose is replaced by 2'-O-methyl ribose in all of the nucleotides in D-stem strand 1, D-stem strand 2 and A-stem strand 1.

7. An RNA molecule according to any one of claims 5-6, wherein all of the ribose is replaced by 2'-O-methyl ribose also in the bridge of 1 - 3 bases.

8. An RNA molecule according to any one of claims 4-7 as set out in SEQ ID NO:2, which molecule interacts with HIV-1 and HIV-2 reverse transcriptase.

9. An RNA molecule according to any one of claims 4-7 as set out in SEQ ID NO:3, which molecule interacts with HIV-1 and HIV-2 reverse transcriptase.

10. An RNA molecule according to any one of claims 5-7 as set out in SEQ ID NO:4, which molecule interacts with HIV-1 and HIV-2 reverse transcriptase.

11. An RNA molecule according to any one of claims 5-7 as set out in SEQ ID NO: 5, which molecule interacts with HIV-1 and HIV-2 reverse transcriptase.

12. A nucleic acid molecule according to any one of claims 1 - 11 for use as a medicament.

13. A nucleic acid molecule according to claim 12 for use as a medicament for the inhibition of the interaction of HIV-1 and HIV-2 reverse transcriptase with tRNA^{Lys3}.

14. Use of a nucleic acid molecule according to any one of claims 1 - 11 for the manufacture of a medicament for the inhibition of the interaction of HIV-1 and HIV-2 reverse transcriptase with tRNA^{Lys3}.

15. A nucleic acid molecule according to any one of claim 1-7 as set out in SEQ ID NO:6, which molecule interacts with HTLV-1 and HTLV-2 reverse transcriptase and corresponds to bases no. 10 - 43 in tRNA^{Pro}.

16. A nucleic acid molecule according to claim 15 for use as a medicament.

## Patentansprüche

1. Synthetisches Nucleinsäuremolekül, das mit reverser Transkriptase eines Retrovirus in Wechselwirkung tritt, wobei die Nucleotidsequenz des Moleküls aus zwei Stamm-Schleife-Strukturen und einer kurzen Brücke zwischen den beiden Stämmen zusammengesetzt ist und das Molekül für die Zwecke der Wechselwirkung mit reverser Transkriptase analog zur Dihydrouridin- (D-) Stamm-Schleife und Anticodon- (A-) Stamm-Schleife einer Säuger-Transfer-RNA (tRNA) ist, indem es die folgenden Strukturelemente umfasst: Strang 1 des D-Stammes, D-Schleife, Strang 2 des D-Stammes, eine Brücke aus einer bis drei Basen, Strang 1 des A-Stamms, A-Schleife, Strang 2 des A-Stammes; und wobei im Molekül wahlweise einige oder alle der normalen Phosphodiester-Nucleosid-Verknüpfungen durch Phosphorothioatverknüpfungen ersetzt worden sind.

2. Nucleinsäuremolekül nach Anspruch 1, **dadurch gekennzeichnet, dass** die Sequenz analog zu einer Säuger-tRNA vom 5'-Ende her die folgende Struktur besitzt: Strang 1 des D-Stamms, D-Schleife, Strang 2 des D-Stamms, eine Brücke aus einer bis drei Basen, Strang 1 des A-Stammes, A-Schleife, Strang 2 des A-Stammes.

3. Nucleinsäuremolekül nach Anspruch 2, **dadurch gekennzeichnet, dass** die Sequenz weiter eine mit dem Ende des Stranges 2 des A-Stamms verknüpfte, ungepaarte Base enthält.

4. Nucleinsäuremolekül nach einem der vorangehenden Ansprüche, das ein RNA-Molekül ist.

5. RNA-Molekül nach Anspruch 4, **dadurch gekennzeichnet, dass** in zumindest einem der Nucleotide in Strang 1 des D-Stamms, Strang 2 des D-Stamms und Strang 1 des A-Stammes die Ribose durch 2'-*O*-Methylribose ersetzt ist.

6. RNA-Molekül nach Anspruch 5, **dadurch gekennzeichnet, dass** in allen Nucleotiden in Strang 1 des D-Stammes, Strang 2 des D-Stammes und Strang 1 des A-Stammes die gesamte Ribose durch 2'-*O*-Methylribose ersetzt ist.

7. RNA-Molekül nach einem der Ansprüche 5 bis 6, **dadurch gekennzeichnet, dass** die gesamte Ribose auch in der Brücke aus einer bis drei Basen durch 2'-*O*-Methylribose ersetzt ist.

8. RNA-Molekül nach einem der Ansprüche 4 bis 7, wie in SEQ ID NO: 2 dargelegt, wobei das Molekül mit reverser Transkriptase von HIV-1 und HIV-2 in Wechselwirkung tritt.

9. RNA-Molekül nach einem der Ansprüche 4 bis 7, wie in SEQ ID NO: 3 dargelegt, wobei das Molekül mit reverser Transkriptase von HIV-1 und HIV-2 in Wechselwirkung tritt.

10. RNA-Molekül nach einem der Ansprüche 5 bis 7, wie in SEQ ID NO: 4 dargelegt, wobei das Molekül mit reverser Transkriptase von HIV-1 und HIV-2 in Wechselwirkung tritt.

11. RNA-Molekül nach einem der Ansprüche 5 bis 7, wie in SEQ ID NO: 5 dargelegt, wobei das Molekül mit reverser Transkriptase von HIV-1 und HIV-2 in Wechselwirkung tritt.

12. Nucleinsäuremolekül nach einem der Ansprüche 1 bis 11 zur Verwendung als ein Medikament.

13. Nucleinsäuremolekül nach Anspruch 12 zur Verwendung als ein Medikament zur Hemmung der Wechselwirkung von reverser Transkriptase von HIV-1 und HIV-2 mit tRNA^{Lys3}.

14. Verwendung eines Nucleinsäuremoleküls nach einem der Ansprüche 1 bis 11 zur Herstellung eines Medikaments zur Hemmung der Wechselwirkung von reverser Transkriptase von HIV-1 und HIV-2 mit tRNA^{Lys3}.

15. Nueleinsäuremolekül nach einem der Ansprüche 1 bis 7, wie in SEQ ID NO: 6 dargelegt, wobei das Molekül mit reverser Transkriptase von HTLV-1 und HTLV-2 in Wechselwirkung tritt und den Basen Nr. 10 bis 43 in tRNA^{Pro} entspricht.

16. Nucleinsäuremolekül nach Anspruch 15 zur Verwendung als ein Medikament.

## Revendications

1. Molécule d'acide nucléique synthétique, qui interagit avec la transcriptase inverse d'un rétrovirus, la séquence nucléotidique de ladite molécule étant composée de deux structures tiges-boucles et d'un pont court entre les deux tiges, laquelle molécule, dans un but d'interaction avec la transcriptase inverse, est analogue à la tige-boucle-dihydrouridine(D) et tige-boucle-antieodon(A) d'un ARN de transfert (ARNt) de mammifère, comprenant les éléments structuraux suivante : brin 1 à tige D, boucle D, brin 2 à tige D, un pont de 1 à 3 bases, brin 1 à tige A, boucle A, brin 2 à tige A ; et éventuellement dans laquelle molécule certains ou la totalité des liaisons normales phosphodiestere des nualéosides ont été substituées par des liaisons phosphorothioates.

2. Molécule d'acide nucléique selon la revendication 1, dans laquelle la séquence, en analogie avec un ARNt de mammifère, a la structure suivante en partant de l'extrémité 5' : brin 1 à tige D, boucle D, brin 2 à tige D, un pont de 1 à 3 bases, brin 1 à tige A, boucle A, brin 2 à tige A.

3. Molécule d'acide nucléique selon la revendication 2, dans laquelle la séquence contient en plus une base non appariée liée à l'extrémité du brin 2 à tige A.

4. Molécule d'acide nucléique selon l'une quelconque des revendications précédentes, qui est une molécule d'ARN.

5. Molécule d'ARN selon la revendication 4, dans laquelle le ribose est remplacé par le 2'-O-méthyl ribose dans au moins un des nucléotides dans le brin 1 à tige D, le brin 2 à tige D et le brin 1 à tige A.

6. Molécule d'ARN selon la revendication 5, dans laquelle tout le ribose est remplacé par du 2'-O-méthyl ribose dans tous les nuoléotides dans le brin 1 à tige D, le brin 2 à tige D et le brin 1 à tige A.

7. Molécule d'ARN selon l'une quelconque des revendications 5 à 6, dans laquelle tout le ribose est remplacé par du 2'-O-méthyl ribose, même dans le pont de 1 à 3 bases.

8. Molécule d'ARN selon l'une quelconque des revendications 4 à 7 comme établie dans SEQ ID N°2, laquelle molécule interagit avec la transcriptase inverse de VIH-1 et VIH-2.

9. Molécule d'ARN selon l'une quelconque des revendications 4 à 7 comme établie dans SEQ ID N°3, laquelle molécule interagit avec la transcriptase inverse de VIH-1 et VIH-2.

10. Molécule d'ARN selon l'une quelconque des revendications 5 à 7 comme établie dans SEQ ID N°4, laquelle molécule interagit avec la transcriptase inverse de VIH-1 et VIH-2.

11. Molécule d'ARN selon l'une quelconque des revendications 5 à 7 comme établie dans SEQ ID N°5, laquelle molécule interagit avec la transcriptase inverse de VIH-1 et VIH-2.

12. Molécule d'acide nucléique selon l'une quelconque des revendications 1 à 11 pour utilisation comme un médicament.

13. Molécule d'acide nucléique selon la revendication 12 pour utilisation comme un médicament destiné à l'inhibition de l'interaction de la transcriptase inverse de VIH-1 et VIH-2 avec l'ARNt^{Lys3}.

14. Utilisation d'une molécule d'acide nucléique selon l'une quelconque des revendications 1 à 11 pour la fabrication d'un médicament destiné à l'inhibition de l'interaction de la transcriptase inverse de VIH-1 et VIH-2 avec l'ARNt^{Lys3}.

15. Molécule d'acide nucléique selon l'une quelconque des revendications 1 à 7 comme établie dans SEQ ID N°6, laquelle molécule interagit avec la transcriptase inverse de HTLV-1 et HTLV-2 et correspond aux bases N° 10 à 43 de l'ARNt^{Pro}.

16. Molécule d'acide nucléique selon la revendication 15 pour utilisation comme un médicament.
